(19) Europäisches Patentamt
European Patent Office
Office européen des brevets

(11) Veröffentlichungsnummer : **0 150 398**
**B1**

(12) # EUROPÄISCHE PATENTSCHRIFT

(45) Veröffentlichungstag der Patentschrift : **17.08.88**

(51) Int. Cl.⁴ : **C 07 D317/12**, A 61 K 7/46

(21) Anmeldenummer : **84115452.9**

(22) Anmeldetag : **14.12.84**

(54) **Neue Methylphenyldioxolane, deren Herstellung und Verwendung als Riechstoffe sowie diese enthaltende Riechstoffkompositionen.**

(30) Priorität : **11.01.84 DE 3400611**

(43) Veröffentlichungstag der Anmeldung : **07.08.85 Patentblatt 85/32**

(45) Bekanntmachung des Hinweises auf die Patenterteilung : **17.08.88 Patentblatt 88/33**

(84) Benannte Vertragsstaaten : **CH DE FR GB LI NL**

(56) Entgegenhaltungen :
**DE-A- 3 004 661**
**DE-B- 1 176 304**
**DE-B- 2 233 245**
**Beilsteins Handbuch der organischen Chemie, Band 19, 3. und 4. Ergänzungswerk, Seite 235**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Blösl, Siegfried, Dr.**
**Itterstrasse 33**
**D-4000 Düsseldorf 13 (DE)**
Erfinder : **Bruns, Klaus, Prof.Dr.**
**Notburgaweg 6**
**D-4150 Krefeld-Traar (DE)**
Erfinder : **Schaper, Ulf-Armin, Dr.**
**Randstrasse 18a**
**D-4150 Krefeld (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 150 398 B1

**Beschreibung**

Wegen der ungleichmäßigen Verfügbarkeit vieler natürlicher Riechstoffkomponenten und der notwendigen Anpassung an wechselnde modische Geschmacksrichtungen hat die Riechstoffindustrie ständig Bedarf an neuen Riechstoffen, die entweder allein oder in Form von Kompositionen wertvolle Parfüms mit interessanten Duftnoten darstellen. Da auf Grund wenig bekannter Zusammenhänge zwischen Struktur und Riechstoffeigenschaften die gezielte Synthese von Riechstoffen mit gewünschter olfaktorischer Qualität nicht möglich ist, besteht die Aufgabe, solche Verbindungen aufzufinden, die wertvolle Riechstoffeigenschaften aufweisen.

2,2-Dimethyl-4-phenyl-1,3-dioxolan ist eine literaturbekannte Verbindung, die durch Umsetzung von Phenyloxiran mit Aceton und Borfluoridether-Addukt hergestellt wird. Angaben über die geruchlichen Eigenschaften dieser Substanz werden nicht gemacht.

Es wurde nun gefunden, daß 4-Methyl-4-phenyl-1,3-dioxolane der Formel

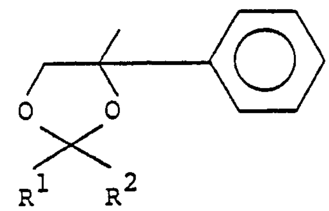

worin bedeuten

$R^1$ = H, Alkyl

$R^2$ = Alkyl, Alkenyl

und $R^1$ und $R^2$ zusammen 1-6 Kohlenstoffatome haben ausgezeichnete neue Riechstoffe sind. Die Verbindungen besitzen eine mittlere Geruchsstärke und eine relativ gute Haftfestigkeit.

Die Substanzen sind bisher nicht bekannt. Ihre Herstellung gelingt nach an sich literaturbekannten Methoden (z. B. R. P. Hanzlik, M. Leinwetter, J. Org. Chem. 43, 438 (1978)) durch Reaktion von α-Methylstyroloxid mit Aldehyden oder Ketonen entsprechend der oben angegebenen Definition von $R^1$, $R^2$ nach folgendem Schema :

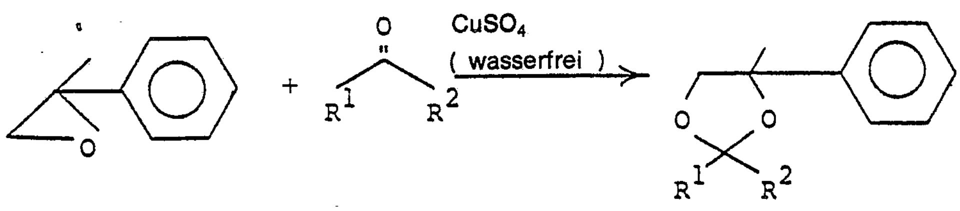


Die Geruchseigenschaften der beanspruchten Verbindungen sind als blumig, süß, Zibet-artig zu beschreiben. Von besonderem Interesse sind das 2(1-Methyl-butyl)-4-methyl-4-phenyl-1,3-dioxolan mit folgender Geruchskennzeichnung : süß, Manna, Zibet-Note, sowie das 2-Ethyl-4-methyl-4-phenyl-1,3-dioxolan mit der Geruchskennzeichnung : Gardenia-, Styrolyl-Note.

In Verbindung mit anderen Riechstoffen und/oder gängigen Parfümbestandteilen lassen sich die neuen Verbindungen zu neuen, interessanten Riechstoffkompositionen kombinieren. Dabei werden etwa 1-50 Gew.-% der neuen Verbindungen, bezogen auf die gesamte Komposition, eingesetzt. Derartige Kompositionen können zur Parfümierung von Kosmetika, wie Duftwässern, Cremes, Lotionen, Aerosolen, Toilettenseifen, in der Extrait-Parfümerie, sowie zur Geruchsverbesserung von technischen Artikeln, wie Wasch- und Reinigungsmitteln, Desinfektionsmitteln, Textilbehandlungsmitteln und dergleichen dienen. Zur Parfümierung der verschiedenen Produkte werden diesen die Kompositionen in Mengen von 0,05-2 Gew.-%, bezogen auf das gesamte Produkt, zugesetzt.

## Beispiele

A. Herstellung von Methylphenyldioxolanen

Allgemeine Arbeitsvorschrift

0,2 Mol Aldehyd oder Keton und 10 Gew.-% $CuSO_4$ (wasserfrei) werden unter Rühren vorgelegt. Bei 50 °C wird innerhalb einer Stunde 7,05 g (0,05 Mol) α-Methylstyroloxid zugetropft. Anschließend läßt man noch 3 Stunden bei 50 °C nachreagieren. Abfiltrieren des Katalysators und nachfolgende fraktionierte Destillation liefert die entsprechenden 1,3-Dioxolane.

1) 2-Ethyl-4-methyl-4-phenyl-1,3-dioxolan
Kp 56 °C/1 mbar $n^{20}_D$ 1,5020
Geruch : Gardenia-, Styrolyl-Note
2) 2(1-Methyl-butyl)-4-methyl-4-phenyl-1,3-dioxolan
Kp 86 °C/1,3 mbar $n^{20}_D$ 1,4892
Geruch : süß, Manna, Zibet-Note
3) 2(But-1-en-2-yl)-4-methyl-4-phenyl-1,3-dioxolan
Kp 89 °C/0,5 mbar $n^{20}_D$ 1,5114
Geruch : Honigbonbon-, Tabaksoße-Note
4) 2(2-Methyl-but-1-en-yl)-4-methyl-4-phenyl-1,3-dioxolan
Kp 103 °C/94 $n^{20}_D$ 1,5120
Geruch : Walnuß-, Maggi-Note, verbrannter Lack
5) 2,4-Dimethyl-2-ethyl-4-phenyl-1,3-dioxolan
Kp 79 °C/1,3 mbar $n^{20}_D$ 1,4961
Geruch : Honig-, Hyacinthe-, Palmarosa-Note

B. Riechstoffkompositionen

| 1. Gardenia-Variante | Gew.-Teile |
|---|---|
| 2-Ethyl-4-methyl-4-phenyl-1,3-dioxolan | 150 |
| Bergamotteöl | 100 |
| Phenylethylalkohol | 100 |
| Benzylacetat | 80 |
| Hydroxycitronellal | 80 |
| Linalool | 60 |
| Phenylmethylcarbinylacetat | 50 |
| Citronellol | 50 |
| Citronellylacetat | 50 |
| Terpineol | 50 |
| α-iron | 30 |
| Cinnamylacetat | 30 |
| Ylang Absolue | 30 |
| Jasmonan ® (Henkel) | 30 |
| Dimethylbenzylcarbinol | 25 |
| Nerol | 20 |
| Isoeugenol | 20 |
| Neroliöl | 15 |
| Geranium Absolue | 15 |
| Sandel ® (H & R) | 10 |
| α-Amylzimtaldehyd | 2 |
| Undecylenaldehyd | 1 |
| Laurinaldehyd | 1 |
| Decylaldehyd | 1 |
| | 1.000 |

| 2. Heliotrop-Komplex | Gew.-Teile |
|---|---|
| 2(1-Methyl-butyl)-4-methyl-4-phenyl-1,3-dioxolan | 150 |
| Heliotropin | 300 |
| Geraniol | 100 |
| Benzylalkohol | 100 |
| Phenylethylalkohol | 60 |
| Ethylvanillin | 55 |
| Farnesol | 30 |
| Zimtalkohol | 30 |
| Hydroxycitronellal | 30 |
| Perubalsamöl | 30 |
| α-Jonon | 30 |
| Bergamotteöl | 20 |
| Cumarin | 20 |
| Anisaldehyd | 20 |
| Bittermandelöl 10 % Benzylalkohol | 10 |
| Vanille Absolue | 5 |

| | |
|---|---|
| Methylnonylacetaldehyd 10 % i. DEP | 5 |
| Nonadienal 1 % i. DEP | 5 |
| | 1.000 |

**Patentansprüche**

1. 4-Methyl-4-phenyl-1,3-dioxolane der Formel

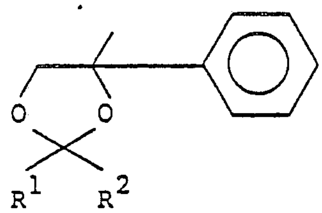

worin bedeuten

$R^1$ = H, Alkyl

$R^2$ = Alkyl, Alkenyl

und $R^1$ und $R^2$ zusammen 1-6 Kohlenstoffatome haben.

2. 2-Ethyl-4-methyl-4-phenyl-1,3-dioxolan.

3. 2(1-Methyl-butyl)-4-methyl-4-phenyl-1,3-dioxolan.

4. Herstellung der 4-Methyl-4-phenyl-1,3-dioxolane entsprechend Anspruch 1 bis 3 durch Reaktion von α-Methylstyroloxid mit Aldehyden oder Ketonen entsprechend der angegebenen Definition von $R^1$, $R^2$ nach folgendem Schema :

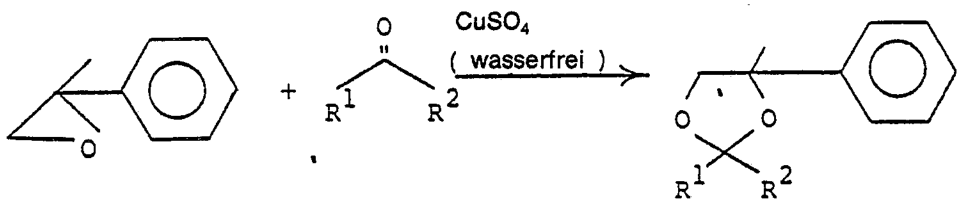


5. Verwendung von 4-Methyl-4-phenyl-1,3-dioxolanen entsprechend Anspruch 1 bis 2 als Riechstoff.

6. Riechstoffkompositionen, gekennzeichnet durch einen Gehalt von 1 bis 50 Gewichtsprozent an 4-Methyl-4-phenyl-1,3-dioxolanen entsprechend Anspruch 1 bis 3.

**Claims**

1. 4-Methyl-4-phenyl-1,3-dioxolanes corresponding to the following formula

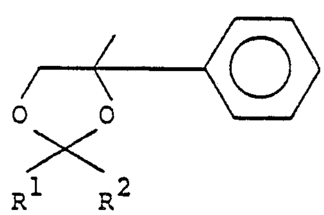

in which

$R^1$ represents H, alkyl,

$R^2$ represents alkyl, alkenyl,

the sum of $R^1$ and $R^2$ amounting to between 1 and 6 carbon atoms.

2. 2-Ethyl-4-methyl-4-phenyl-1,3-dioxolane

3. 2-(1-methylbutyl)-4-methyl-4-phenyl-1,3-dioxolane.

4. A process for producing the 4-methyl-4-phenyl-1,3-dioxolanes claimed in Claims 1 to 3 by reacting α-methyl styrene oxide with aldehyde or ketones corresponding to the definition of $R^1$ and $R^2$ in accordance with the following scheme :

(See scheme page 5)

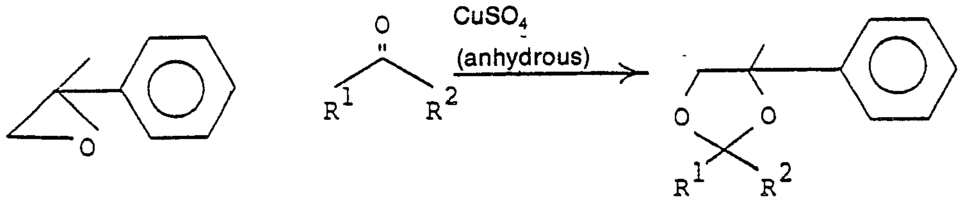


5. The use of the 4-methyl-4-phenyl-1,3-dioxolanes claimed in Claims 1 and 2 as perfumes.

6. Perfume compositions containing from 1 to 50 % by weight of the 4-methyl-4-phenyl-1,3-dioxolanes claimed in Claims 1 to 3.

**Revendications**

1. 4-méthyl-4-phényl-1,3-dioxolane de formule

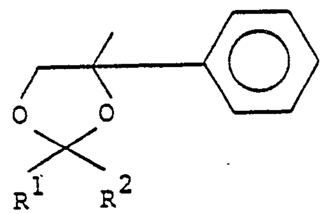

dans laquelle

$R^1$ signifie H, alcoyle

$R^2$ signifie alcoyle, alcényle

et $R^1$ et $R^2$ ensemble ont de 1 à 6 atomes de carbone.

2. 2-éthyl-4-méthyl-4-phényl-1,3-dioxolane.

3. 2(1-méthyl-butyl)-4-méthyl-4-phényl-1,3-dioxolane.

4. Préparation des 4-méthyl-4-phényl-1,3-dioxolanes correspondants aux revendications 1 à 3 par réaction de l'oxyde d'α-méthylstyrène avec des aldéhydes ou des cétones correspondant à la définition indiquée pour $R^1$, $R^2$ selon le schéma suivant :

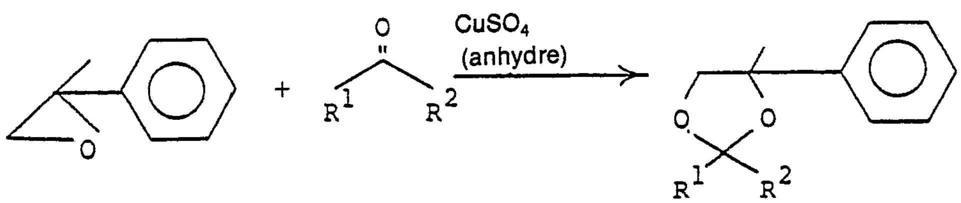


5. Utilisation des 4-méthyl-4-phényl-1,3-dioloxanes correspondant à l'une des revendications 1 ou 2 comme substance aromatique.

6. Compositions de parfum caractérisées par une teneur de 1 à 50 % en poids de 4-méthyl-4-phényl-1,3-dioxolane correspondant aux revendications 1 à 3.